# EUROPÄISCHE PATENTSCHRIFT

(11) **EP 0 696 438 B1**
(45) Veröffentlichungstag und Bekanntmachung des Hinweises auf die Patenterteilung: **05.04.2000**
(21) Anmeldenummer: 95107316.2
(22) Anmeldetag: 15.05.1995
(51) Int. Cl.: A61B 17/58, B23D 23/04, B23D 29/02

(54) **Trennung von Körpern mit nicht kreisrunden Querschnitt**
Severing bodies with non-circular cross sections
Coupage de corps à sections non-circulaires

(30) Priorität: 08.08.1994 CH 245794
(43) Veröffentlichungstag der Anmeldung: 14.02.1996
(73) Patentinhaber: Synthes AG, Chur, 7002 Chur (CH)
(72) Erfinder: Bauer, Peter, CH-4054 Basel (CH); Happe, Herbert, D-79108 Freiburg (DE)
(74) Vertreter: Lusuardi, Werther Giovanni, Dr.

(56) Entgegenhaltungen:
- DE-A- 2 261 281
- DE-C- 4 308 319

## Beschreibung

Die Erfindung bezieht sich auf ein Werkzeug zur Trennung von Körpern gemäss dem Oberbegriff des Patentanspruchs 1, ein Verfahren zur Trennung solcher Körper gemäss dem Oberbegriff des Patentanspruchs 8, sowie einem nach diesem Verfahren hergestellten Körper.

Zur Trennung von osteosynthetischen Platten in der Chirurgie kommt hauptsächlich ein Schneidverfahren mit einem Schneidwerkzeug zur Anwendung. Ein solches Werkzeug ist beispielsweise aus der DE-C1 43 08 311 bekannt. Dabei sind erhebliche Kräfte für das Durchschneiden der osteosynthetischen Platte aufzubringen. Insbesondere sind die durch solche bekannte Schneidzangen aufzubringenden Kräfte sehr gross und die Schneidkanten der Knochenplatten werden verformt oder sind mit einem Grat versehen.

Ein weiteres Verfahren sowie eine Vorrichtung zum gratfreien Trennen von Teilen, insbesondere von Draht- oder Stangenmaterial sind aus der DE 2 261 281 MARTIN bekannt. Dieses bekannte Verfahren beinhaltet das gratfreie Trennen von Stangenmaterial, welches im Bereich der Trennstelle eingespannt ist, durch tordierendes Abscheren. Die zur Durchführung dieses Verfahrens einzusetzende Vorrichtung umfasst Spannstücke, innerhalb welcher die zu bearbeitenden Teile des zu trennenden Materials unmittelbar beiderseits der Trennstelle festgelegt sind. Dabei ist mindestens eines der Spannstücke um die Trennmittellinie drehbar. Die Spannstücke sind als Spannzangen ausgeführt und in Spannkegeln angeordnet, welche ihrerseits auf Widerlagern verschiebbar sind. Das Anziehen der Spannzangen erfolgt mittels einer Überwurfmutter, welche über den Spannkegel gezogen ist.

Daher liegt der vorliegenden Erfindung die Aufgabe zugrunde, ein Verfahren und ein Werkzeug anzugeben, das mit wenig Kraftaufwand die osteosynthetische Platte durchtrennt und eine sauber, nicht verformte Trennstelle gewährleistet.

Die Erfindung löst die gestellte Aufgabe mit einem Werkzeug, welches die Merkmale des Anspruchs 1 aufweist, sowie einem Verfahren, welches die Merkmale des Anspruchs 8 aufweist.

Die zu trennenden Körper können beliebige nicht-kreisrunde Querschnitte aufweisen, d.h. einen prismatischen oder zylindrischen Querschnitt. Im folgenden wird das Verfahren aber lediglich anhand von osteosynthetischen Knochenplatten mit annähernd rechteckigem Querschnitt beschrieben. Vorzugsweise besitzen solche Platten in Abständen ihrer Längsachse Trennfugen, in denen das Werkzeug für den Trennvorgang angesetzt werden kann.

Das Werkzeug besteht aus einem oberen und einem unteren Abscherteil, deren Trennkanten direkt an der Trennkante des jeweils anderen Abscherteils und eng an den Längsflächen der zu trennenden Platte anliegen und durch Verdrehen der beiden Abscherteile wird die Platte getrennt.

Die Erfindung hat den wesentlichen Vorteil, dass die Körper mit erheblich geringerem Kräfteaufwand leicht getrennt werden können und dass ausserhalb der Trennstelle keine Verformung auftritt. Ausserdem ist der Trennvorgang innerhalb kurzer Zeit mit sehr einfachen Hilfsmitteln ausführbar.

Weitere vorteilhafte Ausgestaltungen der Erfindung ergeben sich aus den abhängigen Ansprüchen.

Die Erfindung und Weiterbildungen der Erfindung werden im folgenden anhand der teilweise schematischen Darstellungen mehrerer Ausführungsbeispiele noch näher erläutert.

Es zeigen:
- Fig. 1a: Osteosyntheseplatte mit angesetztem schematisch dargestelltem Werkzeug;
- Fig. 1b: Torsionsdrehung des schematisch dargestellten Werkzeugs;
- Fig. 1c: Osteosyntheseplatte mit abgehobenem oberen Abscherteil;
- Fig. 2a: Zwei scheibenförmige Rundprofile mit Schlitz und Hebelarm;
- Fig. 2b: Osteosyntheseplatte mit angesetztem Werkzeug;
- Fig. 2c: Osteosyntheseplatte mit angesetzten Werkzeugen während des Trennvorgangs;
- Fig. 3: Zwei Zangen;
- Fig. 4: Ein Halter mit Führungsring und scheibenförmigem Profil
- Fig. 5: Werkzeug bestehend aus zwei Abscherteilen.

Die Figur la zeigt mit 1 den oberen Abscherteil und mit 2 den unteren Abscherteil. Jedes Abscherteil weist eine Trennkante 6 auf, die direkt an der Trennkante des jeweils anderen Abscherteils und möglichst eng an den Längsflächen 7 der zu trennenden Platte 3 anliegt. Die Platte 3 weist Trennfugen 9 auf, die in die Trennkanten 6 eingreifen. Ausserdem ist die in ihrem Querschnitt im wesentlichen rechteckige Platte 3 mit Aussparungen 10 versehen, zum Durchstecken von Osteosyntheseschrauben.

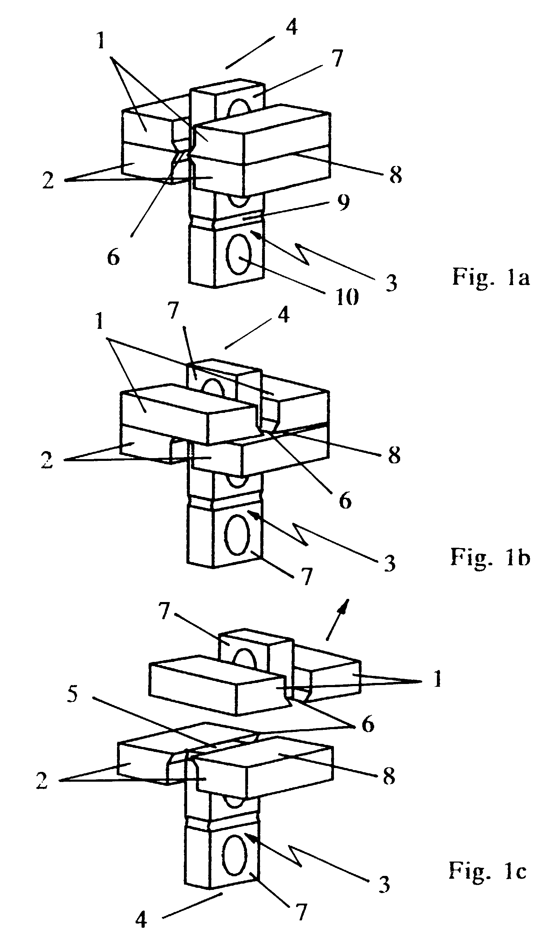

Figur 1b zeigt die Anordnung nach Fig. 1a nach einer Verdrehung der beiden Abscherteile 1 und 2 um mehr als 15° um die vom zu trennenden Material vorgegebene Längsachse 4, die annähernd senkrecht auf der Verdrehebene 8 der Abscherteile steht.

Die Figur 1c zeigt das Separieren der beiden Bestandteile der Platte 3 nach dem erfindungsgemässen Torsionstrennverfahren, und eine für das Trennverfahren charakteristische Trennstelle 5.

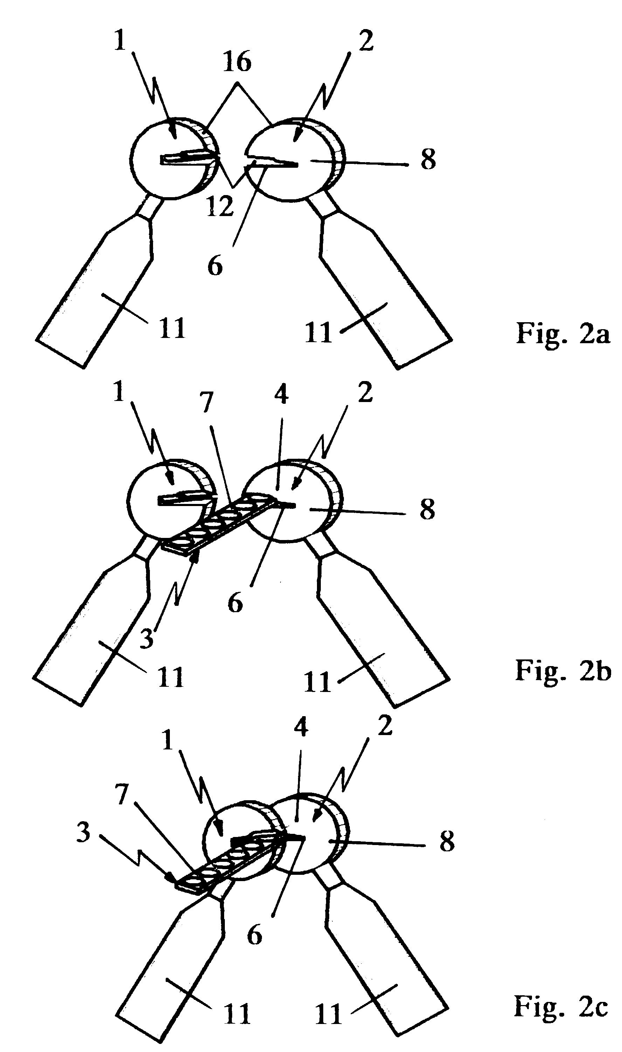

In Figur 2a ist mit 1 und 2 wieder das obere und untere Abscherteil dargestellt, die in dieser Modifikation aus einem scheibenförmigen Profil 16 hervorgehen. Die Trennkanten 6 bestehen aus einem sich in Richtung Zentrum des scheibenförmigen Profils 16 verjüngenden Schlitz 12. Zur Ausübung der Torsionskraft sind Hebelarme 11 vorgesehen.

In Figur 2b ist die Platte 3 in den als Trennkante 6 fungierenden Schlitz 12 des unteren Abscherteils 2 eingeführt. Die Längsachse 4 steht wieder annähernd senkrecht auf der Verdrehebene 8.

Im Beispiel der Figur 2c sind beide Abscherteile 1,2 flächig aneinanderliegend positioniert. Ein gegensinniges Verdrehen der beiden Hebelarme 11 führt zur erfindungsgemässen Torsionstrennung.

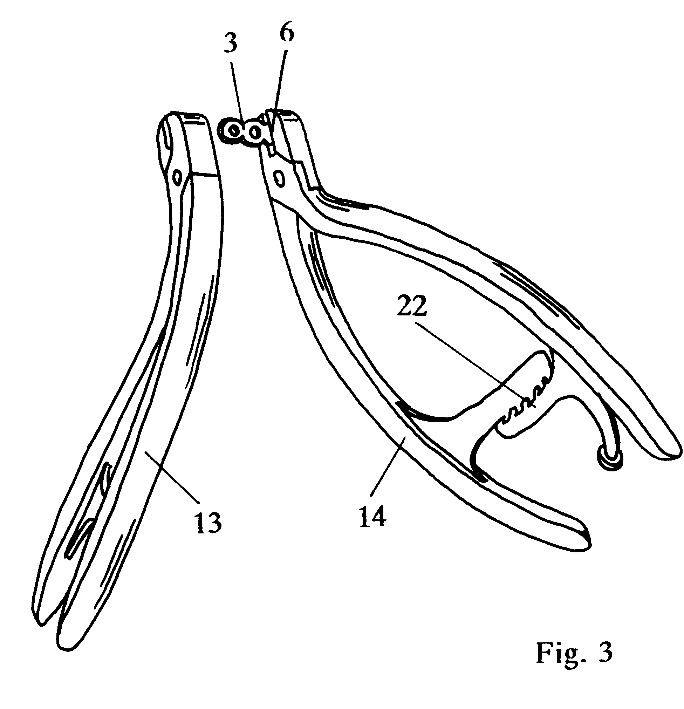

Die in Figur 3 gezeigten Zangen 13, 14 greifen mit ihren Trennkanten 6 in die nicht dargestellten Trennfugen der Platte 3, wobei die Trennkanten 6 wieder direkt aneinander an den Längsflächen der Platte 3 zur Anlage zu bringen sind. Eine gegensinnige Bewegung der gespannten Zangen 13, 14 führt zur gewünschten Torsionstrennung, wobei in der Regel eine mechanische Haltevorrichtung 22 an den Handgriffen das Öffnen der Zange während der Drehung verhindert.

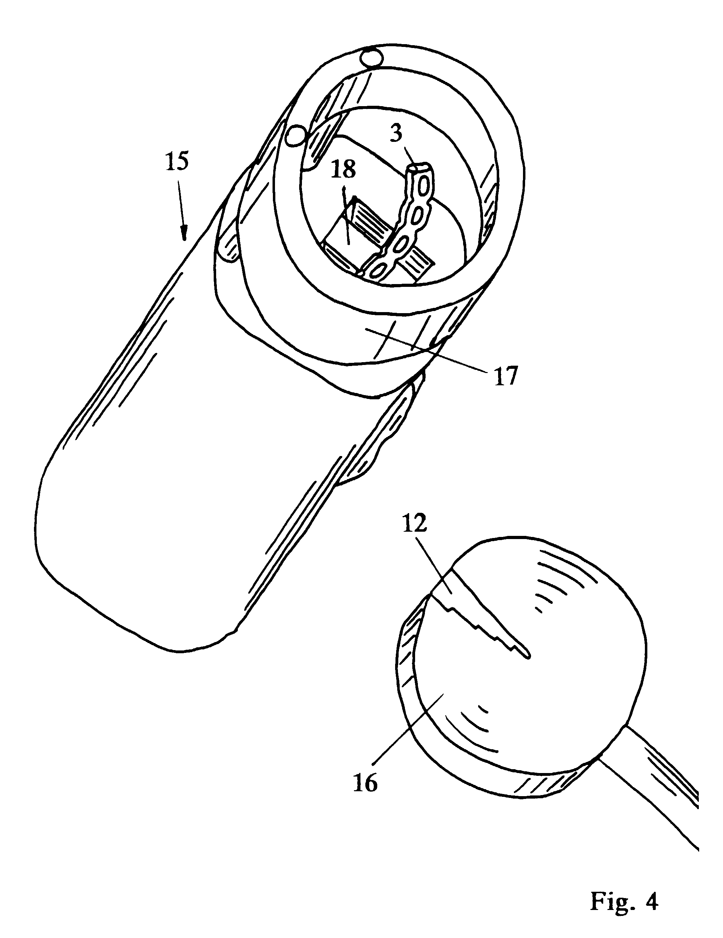

Figur 4 zeigt mit 15 einen Halter mit einem Führungsring 17 und einer Klemmvorrichtung 18, durch die die Platten 3 im Halter 15 gespannt sind und die als ein Abscherteil fungiert. Das andere Abscherteil ist wieder ein scheibenförmiges Profil 16 mit Schlitz 12 wie in den Figuren 2a, b, c beschrieben. Das Rundprofil 16 wird zwischen Halter 15 und Führungsring 17 positioniert, um so eine flächig anliegende Führung zu erreichen, und durch eine gegensinnige Torsionsdrehung werden die Platten 3 getrennt.

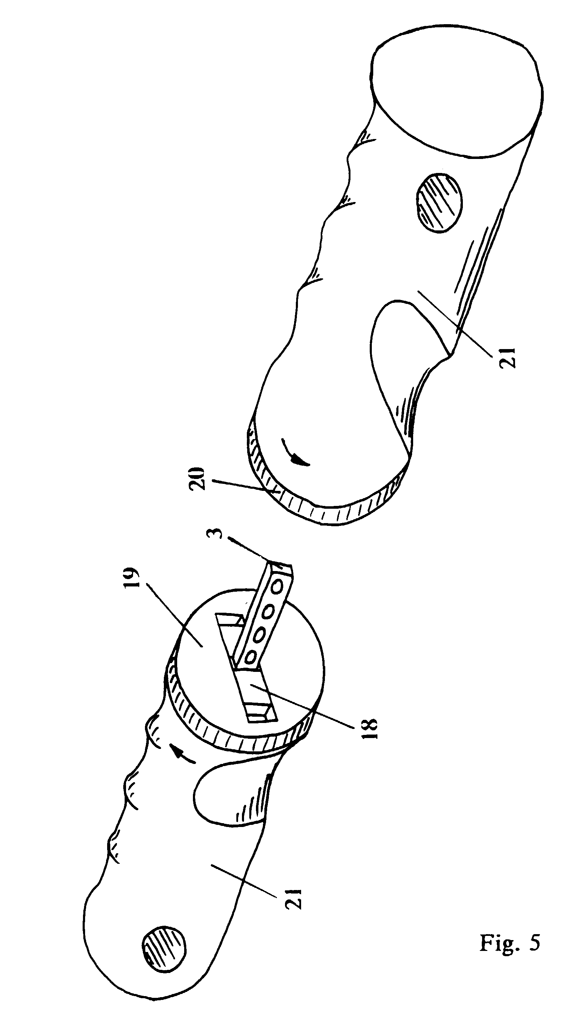

In Figur 5 ist mit 19 ein erstes Abscherteil und mit 20 ein zweites Abscherteil gezeigt mit jeweils den Handgriffen 21. Die Platten 3 werden durch Zuziehen eines Spannmechanismus oder einer Klemmvorrichtung 18 durch eine Drehbewegung des ersten Abscherteils 19 gespannt und das zweite Abscherteil 20 wird mit dem ersten Abscherteil 19 aneinanderliegend positioniert und ebenfalls durch eine Drehbewegung wird das zweite Abscherteil 20 gespannt, so dass durch eine gegenseitige Torsionsdrehung der Handgriffe 21 die Platte 3 getrennt wird.

Während der Drehbewegung sollte zweckmässigerweise mittels einer beliebig gestalteten (zeichnerisch nicht dargestellten) Vorrichtung dafür gesorgt werden, dass die seitlich der Trennkanten 6 liegenden Abscherteile 1,2 sich jeweils nicht voneinander wegbewegen können. Mittels dieser Vorrichtung lässt sich auch das zur Trennung des Körpers erforderliche Drehmoment durch manuellen oder maschinellen Antrieb an die Abscherteile 1,2 weiterleiten. Grundsätzlich ist es auch möglich, dass der Abscherteil 1 oder 2 direkter Bestandteil einer solche Vorrichtung ist.

## Patentansprüche

1. Werkzeug zur Trennung von Körpern, wobei das Werkzeug aus einem oberen Abscherteil (1) und einem unteren Abscherteil (2) besteht, deren Trennkanten (6) an eine bezüglich des oberen und unteren Abscherteils (1;2) gemeinsame Verdrehebene (8) anstossen, so dass die Trennkanten (6) direkt aneinanderliegen und die Distanz zwischen den Trennkanten (6) an jedem Abscherteil (1;2) variabel ist, so dass die Trennkanten (6) eng an die Längsflächen (7) des zu trennenden Körpers (3) zur Anlage bringbar sind und durch Verdrehen der beiden Abscherteile (1;2) um eine durch die Berührungsstellen der Trennkanten (6) mit dem Körper (3) vorgegebene Rotationsachse, die annähernd senkrecht auf der Verdrehebene (8) der Abscherteile (1;2) steht, den Körper (3) trennt,
dadurch gekennzeichnet, dass
das Werkzeug Hebelarme (11), Halter (15) oder Handgriffe (21) umfasst, in der Weise, dass das Werkzeug mittels dieser Hebelarme (11), Halter (15) oder Handgriffe (21) in-situ von Hand haltbar und bedienbar ist.

2. Werkzeug nach Anspruch 1, dadurch gekennzeichnet, dass die Abscherteile (1;2) aus zwei scheibenförmigen Profilen (16) bestehen, an denen jeweils ein Hebelarm (11) angebracht ist, und dass die Profile (16) mit jeweils einem in Richtung des Zentrums sich verjüngenden Schlitz (12) als Trennkanten (6) versehen sind, in den die Körper (3) einführbar sind.

3. Werkzeug nach Anspruch 1, dadurch gekennzeichnet, dass die Abscherteile (1;2) aus zwei scheibenförmigen Profilen (16) bestehen, an denen jeweils ein Hebelarm (11) angebracht ist, und dass die Profile (16) mit einem oder mehreren Ausschnitten als Trennkanten (6) versehen sind, in die der zu trennende Körper (3) einführbar ist.

4. Werkzeug nach Anspruch 1, dadurch gekennzeichnet, dass zwei Zangen (13;14) vorgesehen sind, die mit ihren Schneidkanten in die Trennfugen (9) in der Verdrehebene (8) in Eingriff bringbar sind, um durch eine Torsionsbewegung der beiden Zangen (13;14) die Körper (3) zu trennen.

5. Werkzeug nach Anspruch 1, dadurch gekennzeichnet, dass dieses einen Halter (15) mit einem Führungsring (17) und einer Klemmvorrichtung (18) zum Einspannen eines Körpers (3) sowie ein scheibenförmiges Profil (16) mit Hebelarm (11) und Schlitz (12) umfasst, wobei das scheibenförmige Profil (16) um die Trennachse drehbar zwischen den Halter (15) und den Führungsring (17) bringbar ist, so dass ein in der Klemmvorrichtung (18) eingespannter Körper (3) durch relatives Verdrehen von Halter (15) und scheibenförmigem Profil (16) mittels der durch den Schlitz (12) gebildeten Trennkanten trennbar ist.

6. Werkzeug nach Anspruch 1, dadurch gekennzeichnet, dass das Werkzeug aus einem ersten Abscherteil (19) und einem zweiten Abscherteil (20) mit jeweils Handgriffen (21) besteht, wobei die Abscherteile (19;20) je einen Spannmechanismus, welcher durch eine Drehbewegung der Abscherteile (19;20) bedienbar ist, zum lösbaren Festklemmen eines Körpers (3) umfassen.

7. Werkzeug nach einem der Ansprüche 1 - 6, dadurch gekennzeichnet, dass zwischen den Handgriffen (21) eine mechanische Haltevorrichtung (22) das Öffnen des Werkzeugs während der Torsionsdrehung verhindert.

8. Verfahren zur Trennung von zylindrischen oder prismenförmigen Körpern (3) mit nicht kreisrundem Querschnitt, dadurch gekennzeichnet, dass mittels eines Werkzeugs nach einem der Ansprüche 1 bis 7 eine Torsion auf den Körper (3) ausgeübt wird.

9. Verfahren nach Anspruch 8, dadurch gekennzeichnet, dass als Körper (3) osteosynthetische Platten für die Chirurgie Anwendung finden.

10. Verfahren nach Anspruch 8 oder 9, dadurch gekennzeichnet, dass das Werkzeug in die in Abständen ihrer Längsachse auf den Körpern (3) vorhandenen Trennfugen (9) angesetzt wird.

11. Verfahren nach Anspruch 10, dadurch gekennzeichnet, dass das Werkzeug aus im wesentlichen zwei Vorrichtungen besteht, die an den Trennfugen (9) angrenzend flächig aneinander positioniert werden und durch Verdrehen beider Vorrichtungen gegeneinander die Körper (3) durchtrennen.

12. Verfahren nach Anspruch 10, dadurch gekennzeichnet, dass die Drehachse, um die die Torsionsdrehung erfolgt, annähernd durch die Längsachse des zu trennenden Körpers vorgegeben ist.

13. Zylindrischer oder prismenförmiger Körper mit nicht kreisrundem Materialquerschnitt, hergestellt gemäss dem Verfahren nach einem der Ansprüche 8 bis 12.

## Claims

1. Tool for cutting of bodies, whereby the tool consists of an upper shearing element (1) and a lower shearing element (2) whose cutting edges (6) abut on a plane of rotation (8) which is mutual with respect to the upper and lower shearing element (1;2), such that the cutting edges (6) are in direct contact with each other and the distance between the cutting edges (6) at each shearing element (1;2) is variable so that the cutting edges (6) are able to be brought in close contact with the longitudinal areas (7) of the body to be cut and through rotating the two shearing elements (1;2) relative to one another and with respect to an rotational axis formed through the contact points between the cutting edges (6) and the body (3), which is approximately perpendicular to the plane of rotation (8) of the shearing elements (1;2), separates the body,
characterized in that
the tool comprises lever arms (11), holder (15) or manual grips (21), such that the tool is able to be hand-held and operated by means of these lever arms (11), holder (15) or manual grips (21).

2. Tool according to claim 1, characterized in that the shearing elements (1;2) consist of two disc-shaped units (16) whereon a lever arm (11) is attached each and that as cutting edges (6) the disc-shaped units (16) are provided with slots (12) converging toward the center, wherein the bodies (3) are insertable.

3. Tool according to claim 1, characterized in that the shearing elements (1;2) consist of two disc-shaped units (16) whereon a lever arm (11) is attached each and that as cutting edges (6) the disc-shaped units (16) are provided with one or more cutouts, wherein the bodies (3) are insertable.

4. Tool according to claim 1, characterized in that two forceps (13;14) are provided, that are able to be engaged with their cutting edges into the dividing lines 9 in the plane of rotation (8) to cut the bodies (3) through a torsional motion of the two forceps (13;14).

5. Tool according to claim 1, characterized in that it comprises a holder (15) with a guide ring (17) and with a clamping unit (18) to clamp a body (3) as well as a disc-shaped unit (16) with a lever arm (11) and slot (12), whereby the disc-shaped unit (16) is able to be inserted rotatable around the separation axis between the holder (15) and the guide ring (17), so that a body (3) clamped within the clamping unit (18) is separable through relative rotation of holder (15) and disc-shaped unit (16) by means of the cutting edges formed by the slot (12).

6. Tool according to claim 1, characterized in that the tool consists of a first shearing element (19) and a second shearing element (20) with manual grips (21) each, whereby the shearing elements (19;20) each comprise a clamping mechanism which is able to loosenably clamp a body (3) and is able to be operated via a turning motion of the shearing elements (19;20).

7. Tool according to one of the claims 1 - 6, characterized in that a mechanical holding device (22) between the manual grips (21) prevents the tool from opening during torsional motion.

8. Method for cutting of cylindrical or prismatic bodies (3) having a non-circular cross-section, characterized in that by means of a tool according to one of the claims 1 to 7 a torsional force is applied to the body (3).

9. Method according to claim 8, characterized in that as bodies (3) osteosynthetic plates for surgery are used.

10. Method according to claim 8 or 9, characterized in that the tool is engaged into the dividing lines (9) wherewith the bodies (3) are provided within sections of their longitudinal axes.

11. Method according to claim 10, characterized in that essentially consists of to devices that are positioned surface-to-surface at the dividing lines (9) and separate the bodies (3) by means of a counterturning motion of the devices relative to one another.

12. Method according to claim 10, characterized in that the rotation axis where around the torsional motion is executed is approximately defined through the longitudinal axis of the body to be cut.

13. Cylindrical or prismatic body having a non-circular cross-section produced according to the method according to one of the claims 8 to 12.

## Revendications

1. Outil pour la découpe de corps, l'outil étant constitué d'une pièce supérieure de cisaillage (1) et d'une pièce inférieure de cisaillage (2) dont les arêtes de découpe (6) viennent se rejoindre dans un plan de torsion (8) commun de la pièce de cisaillage supérieure et de la pièce de cisaillage inférieure (1; 2), de telle sorte que les arêtes de découpe (6) viennent se placer directement l'une contre l'autre et que la distance entre les arêtes de découpe (6) de chaque pièce de cisaillage (1; 2) soit variable, de telle sorte que les arêtes de découpe (6) puissent être placées contre les surfaces longitudinales (7) du corps (3) à découper, et qu'elles découpent le corps (3) par rotation des deux pièces de cisaillage (1; 2) autour d'un axe de rotation prédéfini par les emplacements de contact des arêtes de découpe (6) avec le corps (3), lequel axe de rotation est approximativement perpendiculaire au plan de rotation (8) des pièces de cisaillage (1; 2),
caractérisé en ce que l'outil comporte des bras de levier (11), des supports (15) ou des poignées (21), de telle sorte que l'outil puisse être maintenu et utilisé in situ à la main au moyen de ses bras de levier (11), de ses supports (15) ou de ses poignées (21).

2. Outil selon la revendication 1, caractérisé en ce que les pièces de cisaillage (1; 2) sont constituées de deux profils (16) en forme de disque sur chacun desquels est installé un bras de levier (11), et en ce que les profils (16) sont dotés chacun d'une fente (12) qui se rétrécit en direction du centre et qui sert d'arête de découpe (6), dans lesquelles les corps (3) peuvent être insérés.

3. Outil selon la revendication 1, caractérisé en ce que les pièces de cisaillage (1; 2) sont constituées de deux profils (16) en forme de disque sur chacun desquels est installé un bras de levier (11), et en ce que les profils (16) sont dotés d'une ou de plusieurs parties servant d'arête de découpe (6), dans lesquelles le corps (3) à découper peut être inséré.

4. Outil selon la revendication 1, caractérisé en ce que sont prévues deux pinces (13; 14) dont les tranchants de découpe peuvent être engagés dans les entailles de découpe (9) dans le plan de rotation (8), pour découper les corps (3) par un déplacement de torsion des deux pinces (13; 14).

5. Outil selon la revendication 1, caractérisé en ce que celui-ci comporte un support (15) doté d'une bague de guidage (17) et d'un dispositif de pinçage (18) pour serrer un corps (3), et comporte également un profil (16) en forme de disque doté d'un bras de levier (11) et d'une fente (12), le profil (16) en forme de disque pouvant être amené entre le support (15) et la bague de guidage (17) de manière à pouvoir tourner autour de l'axe de découpe, de sorte qu'un corps (3) serré dans le dispositif de pinçage (18) peut être découpé par une rotation relative du support (15) et du profil (16) en forme de disque, au moyen des arêtes de découpe formées par la fente (12).

6. Outil selon la revendication 1, caractérisé en ce que l'outil est constitué d'une première pièce de cisaillage (19) et d'une deuxième pièce de cisaillage (20) qui présentent chacune des poignées (21), et les pièces de cisaillage (19; 20) présentent chacune un mécanisme de serrage qui peut être actionné par un déplacement de rotation des pièces de cisaillage (19; 20) pour le serrage libérable d'un corps (3).

7. Outil selon l'une des revendications 1 - 6, caractérisé en ce qu'entre les poignées (21), un dispositif mécanique de retenue (22) empêche l'ouverture de l'outil pendant la rotation de torsion.

8. Procédé pour la découpe de corps (3) cylindriques ou prismatiques à section transversale non circulaire, caractérisé en ce qu'une torsion est exercée sur le corps (3) au moyen d'un outil selon l'une des revendications 1 à 7.

9. Procédé selon la revendication 8, caractérisé en ce que comme corps (3), on utilise des plaques d'ostéosynthèse pour la chirurgie.

10. Procédé selon la revendication 8 ou 9, caractérisé en ce que l'outil est placé dans des entailles de découpe (9) prévues sur les corps (3) à distance de leur axe longitudinal.

11. Procédé selon la revendication 10, caractérisé en ce que l'outil est constitué essentiellement de deux dispositifs qui sont positionnés l'un contre l'autre en se rejoignant par une surface sur les entailles de découpe (9), et qui découpent les corps (3) par rotations opposées des deux dispositifs.

12. Procédé selon la revendication 10, caractérisé en ce que l'axe de rotation autour duquel s'effectue la rotation de torsion est défini approximativement par l'axe longitudinal du corps à découper.

13. Corps cylindrique ou prismatique dont le matériau présente une section transversale non circulaire, fabriqué par le procédé selon l'une des revendications 8 à 12.
